# NEUE EUROPÄISCHE PATENTSCHRIFT

(11) **EP 0 488 045 B2**
(45) Veröffentlichungstag und Bekanntmachung des Hinweises auf die Entscheidung über den Einspruch: **13.10.1999**
(45) Hinweis auf die Patenterteilung: 01.03.1995
(21) Anmeldenummer: 91119835.6
(22) Anmeldetag: 21.11.1991
(51) Int. Cl.: C07C 49/603, C07C 45/67

(54) **Verfahren zur Herstellung von beta-Isophoron**
Process for the preparation of beta-Isophorone
Procédé pour la préparation de bêta-Isophorone

(30) Priorität: 30.11.1990 CH 379890
(43) Veröffentlichungstag der Anmeldung: 03.06.1992
(73) Patentinhaber: F. HOFFMANN-LA ROCHE AG, 4002 Basel (CH)
(72) Erfinder: Nösberger, Paul, Dr., CH-4127 Birsfelden (CH); Vieth, Adrian James, W-7856 Rümmingen (DE)
(74) Vertreter: Cottong, Norbert A.

(56) Entgegenhaltungen:
- EP-A- 0 312 735
- GB-A- 588 099
- GB-A- 1 265 029
- US-A- 2 183 127
- CHEMICAL ABSTRACTS, Band 112, Nr. 15, 1990, Columbus, Ohio, US; Zusammenfassung Nr. 138649F, N. ITO et al: "Preparation of 3,5,5-trimethylcyclohex-3-en-1-one from 3,5,5-trimethylcyclohex-2-en-1-one as material for pharmaceuticals and fragrances", Seite 682; JP-A-01 175 954
- HOUBEN-WEYL, "Methoden der organischen Chemie" 4/2, Seiten 276-79

## Beschreibung

Die Erfindung bezieht sich auf ein Verfahren zur Herstellung von beta-Isophoron (3,5,5-Trimethylcyclohex-3-en-1-on) aus alpha-Isophoron (3,5,5-Trimethylcyclohex-2-en-1-on) durch Gasphasenisomerisierung.

beta-Isophoron besitzt grosses wirtschaftliches Interesse für die Synthese wichtiger Verbindungsklassen. Insbesondere wird beta-Isophoron als Vorstufe für Ketoisophoron (2,6,6-Trimethylcyclohex-2-en-1,4-dion) und damit für die Herstellung des Vitamins E, verschiedener Carotinoide, Riechstoffe und weiterer Naturprodukte benötigt.

Die Herstellung von Isophoron gelingt durch wasserabspaltende Trimerisation von Aceton. Dabei bildet sich jedoch hauptsächlich das alpha-Isomere in über 90%iger Ausbeute. Das thermodynamische Gleichgewicht des Systems alpha-Isophoron/beta-Isophoron liegt auf der Seite des alpha-Isophorons, weil durch die Verschiebung der Doppelbindung ein energiearmes konjugiertes System in ein energiereicheres nicht-konjugiertes System übergeführt werden muss. Die Einstellung dieses Gleichgewichts erfolgt zudem langsam. Dies bedeutet, dass die Ueberführung von alpha-Isophoron in beta-Isophoron mit Schwierigkeiten verbunden ist.

Von besonderem Interesse ist daher die Isomerisierung des alpha-Isophorons zum beta-Isophoron in hoher Ausbeute, sowie unter wirtschaftlichen Gesichtspunkten.

Es wurden bereits einige Verfahren zur Isomerisierung von alpha-Isophoron in beta-Isophoron beschrieben. Die Isomerisierung ist z.B. möglich durch Umsetzung von alpha-Isophoron mit molaren Mengen Methylmagnesiumjodid unter Zusatz von Eisen(III)-chlorid, anschliessende Hydrolyse und destillative Aufarbeitung [A. Heymes et al., Recherches 1971, 18, 104-108].

Die Isomerisierung kann auch durch mehrstündiges Kochen mit Triäthanolamin, anschliessende Fraktionierung und Waschen des Destillats mit Weinsäure und Kochsalzlösung erfolgen [DE-OS 2457157].

Ein weiterer Zugang zu beta-Isophoron ist die katalytische Isomerisierung von alpha-Isophoron mit wenig dissoziierten Säuren [USP 4.005.145]. Bei diesen Verfahren wird das gebildete beta-Isophoron destillativ aus dem Gleichgewicht entfernt. Der Gehalt an beta-Isophoron in der Destillationsblase beträgt 1-2%.

Nach dem gleichen Prinzip arbeitet ein Verfahren mit Acetylacetonaten oder Metallen der Gruppen IVB, VB, VIB, VIIB und VIIIB des Periodensystems, sowie von Aluminium [EP 312 735].

Alle bekannt gewordenen Verfahren weisen jedoch Nachteile auf, und zwar:.
- Die Einstellung des Gleichgewichtes ist auch mit den besten bisher bekannten Katalysatoren ein relativ langsamer Prozess. Die Raum-/Zeitausbeuten sind entsprechend niedrig.
- Bei einer Reaktionstemperatur über 200°C findet bei der destillativen Abtrennung von beta-Isophoron eine Rückisomerisierung statt. Zur Gewinnung von reinem beta-Isophoron ist eine zweite Destillation bei einem reduzierten Druck notwendig.
- Der Energieaufwand zur destillativen Trennung des Gemisches alpha-Isophoron/beta-Isophoron ist gross, da der Gehalt an beta-Isophoron sehr klein ist.

Als weiterer Stand der Technik beschreibt die UK-Patentschrift 588 099 ein Verfahren zur Herstellung von 3,5-Dimethylphenol aus alpha-Isophoron über das kurzlebige Zwischenprodukt 3,5-Dimethyl-cyclohexa-2,4-dien-1-on durch Abspaltung von Methan im ersten Verfahrensschritt. Diese Umwandlung erfolgt in der Gasphase, vorzugsweise bei Temperaturen von etwa 400°C bis etwa 550°C (die diesbezüglichen Beispiele belegen Reaktionstemperaturen von etwa 500°C bis etwa 700°C), an einem festen Katalysator, welcher hauptsächlich oder ausschliesslich aktiviertes Alumina enthält. Die UK-Patentschrift enthält keinerlei Hinweise auf die Herstellung von beta-Isophoron aus alpha-Isophoron.

Es wurde nun ein Verfahren zur Gewinnung von beta-Isophoron gefunden, das die Nachteile des Standes der Technik weitgehend eliminiert.

Dieses Verfahren beinhaltet die Isomerisierung von alpha-Isophoron zu beta-Isophoron in der Gasphase in Gegenwart eines heterogenen Katalysators.

Gegenstand der Erfindung ist daher ein Verfahren zur Herstellung von beta-Isophoron aus alpha-Isophoron, welches dadurch gekennzeichnet ist, dass alpha-Isophoron in Gegenwart eines heterogenen Katalysators in der Gasphase bei einer Temperatur von 300°C bis 400°C zu beta-Isophoron umgesetzt wird. Als heterogener Katalysator werden Oxide oder Mischoxide zweckmässigerweise der Elemente Mg, Al, Si und Ni verwendet. Als Katalysator kann auch Aluminiumsilikat benutzt werden, wobei dieses als Mischoxid von Aluminium- und Siliciumoxiden angesehen werden soll.

Der Katalysator kann in reiner Form vorliegen, mit einem inerten, insbesondere möglichst wenig porösem, Trägermaterial gemischt sein oder auf einem inerten, geformten Trägermaterial, z.B. Keramik, Glas oder auch γ-Aluminiumoxid, fixiert sein.

Ein bevorzugter Katalysator ist Nickeloxid allein oder Nickeloxid, gegebenenfalls calciniert, auf einem inerten, geformten Trägermaterial (beispielsweise Keramik, Glas sowie auch γ-Aluminiumoxid). Besonders bevorzugt ist Nickeloxid auf einem keramischen Trägermaterial. Ein weiterer besonders bevorzugter Katalysator ist bei einer Temperatur von ≧ 1000°C calciniertes Nickeloxid auf einem γ-Aluminiumoxid-Träger.

Vorzugsweise besitzt der Katalysator eine spezifische Oberfläche von etwa 1-50 m²/g und weist einen möglichst geringen Anteil an Mikroporen und kleinen Mesoporen auf.

Das erfindungsgemässe Verfahren kann vorzugsweise in einem Festbettreaktor durchgeführt werden.

Eine Verdünnung mit Inertgas ist zwar möglich, aber für die Durchführung der Isomerisierungsreaktion unwesentlich. Vorzugsweise wird die Reaktion bei einem Druck von 1 kPa bis 200 kPa durchgeführt. Besonders bevorzugt wird die Reaktion bei Atmosphärendruck durchgeführt. Im allgemeinen sollen die Kontaktzeiten im Bereich von 0,1 Sekunde bis 10 Sekunden liegen, wobei der Bereich von 0,5 Sekunde bis 2 Sekunden bevorzugt ist. Die Zufuhr von alpha-Isophoron beträgt zweckmässigerweise 1-10 kg/h·l. Das heisst, dass pro Liter Katalysator und Stunde geeigneterweise 1-10 kg alpha-Isophoron eingesetzt wird.

Die Optimierung wird zweckmässigerweise so durchgeführt, dass der Katalysator und gewünschtenfalls inertes Trägermaterial in den Reaktor (vorzugsweise ein Festbettreaktor) eingefüllt werden und dann die Verweilzeit und die Temperatur so eingestellt werden, dass der Umsatz an Edukt optimal ist und keine Nebenprodukte gebildet werden.

Die Reaktion wird in einem Temperaturbereich von 300°C bis 400°C durchgeführt.

Gasförmiges alpha-Isophoron, eventuell mit Inertgas verdünnt, wird zweckmässigerweise durch die beheizte, mit dem unverdünnten oder auf einem inerten Trägermaterial fixierten oder damit gemischten Katalysator gefüllte Reaktionszone geleitet. Abhängig von der Reaktionstemperatur enthalten die Reaktionsprodukte 5-11% beta-Isophoron und 89-95% alpha-Isophoron. Nebenprodukte, beispielsweise gamma-Isophoron (3-Methylen-5,5-dimethylcyclohexan-1-on), Kohlenwasserstoffe, 3,5-Xylenol und 2,3,5-Trimethylphenol, werden nur in unbedeutender Menge gebildet. Die Reaktionsprodukte werden auf übliche Art und Weise, beispielsweise destillativ, aufgetrennt.
Besonders attraktiv ist die direkte Zufuhr der Reaktionsprodukte zu einer kontinuierlich betriebenen Rektifikationskolonne, wobei am Kopf beta-Isophoron mit einem Gehalt von ca. 97% anfällt. Das Fussprodukt der Kolonne kann ohne weitere Reinigung zum Reaktor zurückgeführt werden.

### Beispiel 1

Als Katalysator wird granuliertes Nickeloxid (Merck 6723) mit einer Körnung von 1-3 mm verwendet. Zur Granulierung des Nickeloxid-Pulvers wird 1% Luviskol K90 (Polyvinylpyrrolidon) zugegeben. 45 ml dieses Katalysators werden in ein elektrisch geheiztes Glasrohr mit einem Durchmesser von 2,4 cm eingefüllt. Ueber dem Katalysator wird das Glasrohr auf einer Länge von ca. 4 cm mit Glaswolle gefüllt. Bei einer Temperatur von 300°C werden nun stündlich 18 ml alpha-Isophoron und 1,8 l Stickstoff von oben zugeführt. Die Reaktionsprodukte enthalten 9% beta-Isophoron und 91% alpha-Isophoron.

### Beispiel 2

Es wird käufliches Nickeloxid (Strem 28-148; 15% Nickeloxid auf γ-Aluminiumoxid) als Katalysator eingesetzt. Dieses wird vor dem Gebrauch während 3 Stunden bei 1200°C calciniert. Die Apparatur zur Durchführung der Isomerisierung besteht aus einem von aussen beheizbaren Verdampfungskolben mit einem darüber befindlichen, elektrisch von aussen beheizten Reaktionsrohr. Eine Zuleitung führt zu einem Kühler, welcher mit einem Auffanggefäss verbunden ist. Die Isomerisierungsreaktion wird bei einem Druck von 2 kPa durchgeführt.
Im Reaktionsrohr (Durchmesser 5 cm) werden zuerst 45 ml Glaskugeln und dann 100 ml Katalysator eingefüllt. Die Temperatur des Katalysators wird auf 300°C eingestellt. Der Verdampfungskolben wird auf 105°C erhitzt. Stündlich entnimmt man ca. 100 ml Reaktionsprodukte mit einem Gehalt an beta-Isophoron von 6-7%. Das Gemisch enthält weiterhin ca. 93% alpha-Isophoron und ca. 0,3% gamma-Isophoron.

### Beispiel 3

30 ml eines Magnesiumoxid-Katalysators (Mg-0601T von Harshaw) werden in ein elektrisch geheiztes Glasrohr (Durchmesser 2 cm) eingefüllt. Darüber und darunter befinden sich Keramikkugeln. Bei einer Reaktionstemperatur von 300°C werden 4 l Stickstoff pro Stunde und 90 ml alpha-Isophoron pro Stunde zugeführt. Die Reaktionsprodukte bestehen aus 7,6% beta-Isophoron, 91,8% alpha-Isophoron und 0,6% Nebenprodukten.

### Beispiel 4

Anstelle des Magnesiumoxid-Katalysators aus Beispiel 3 wird alpha-Aluminiumoxid (A 980 von Rosenthal) verwendet. Die Reaktion wird analog Beispiel 3 durchgeführt. Das Reaktionsgemisch enthält 6,8% beta-Isophoron. Es lassen sich keine Nebenprodukte nachweisen.

### Beispiel 5

Als Katalysator wird Siliciumdioxid mit einer spezifischen Oberfläche von 50 m²/g (Shell S980G/2,5) verwendet. Der Reaktor besteht aus einem elektrisch beheizten Stahlrohr (geheizte Zone: 43 cm; Durchmesser 2,7 cm). Es werden 40 ml Katalysator eingefüllt. Im unteren und oberen Teil wird der Reaktor mit Keramikkugeln (Durchmesser 0,6 cm) gefüllt. Bei einer Reaktionstemperatur von 300°C werden dem Reaktor stündlich zwischen 60 und 240 ml alpha-Isophoron zugeführt. Das Reaktionsgemisch enthält, unabhängig von der Menge des zugeführten Isophorons, 5,5% beta-Isophoron und 94,5% alpha-Isophoron.

### Beispiel 6

Als Katalysator wird Aluminiumsilikat (Norton SA-3232) mit einer spezifischen Oberfläche von 30 m²/g eingesetzt. Es wird der gleiche Reaktor und dieselbe Katalysatormenge wie in Beispiel 5 verwendet. Dem Reaktor werden stündlich 4 l Stickstoff und 180 ml alpha-Isophoron zugeführt.

Die Ausbeute an beta-Isophoron ist abhängig von der Reaktionstemperatur und in der nachfolgenden Tabelle veranschaulicht.

**Tabelle**

| Abhängigkeit des Gehaltes an beta-Isophoron von der Reaktionstemperatur | | | |
|---|---|---|---|
| **Temperatur °C** | **β-Isophoron %** | **α-Isophoron %** | **Nebenprodukte %** |
| 300 | 5,0 | 95,0 | - |
| 350 | 6,8 | 93,2 | - |
| 400 | 9,2 | 90,8 | - |
| 450 | 11,3 | 84,7 | 4,0 ^{a)} |

| | | | |
|---|---|---|---|
| ^{a)} Die gebildete Menge an Nebenprodukten kann durch Erhöhung der Isophoronzufuhr reduziert werden. Die Angaben für 450°C sind Vergleichsdaten. | | | |

### Beispiel 7

Es wird der gleiche Katalysator und derselbe Reaktor wie in Beispiel 6 verwendet. Es werden 100 ml Katalysator eingesetzt. Die Reaktionstemperatur wird auf 350°C eingestellt und dem Reaktor stündlich 400 g alpha-Isophoron zugeführt. Die Reaktion wird nach 40 Stunden beendet. Das Reaktionsprodukt enthält 7,7% beta-Isophoron. Es werden keine Nebenprodukte nachgewiesen. Die Aktivität des Katalysators bleibt während der Versuchsdauer konstant.

## Patentansprüche

1. Verfahren zur Herstellung von beta-Isophoron aus alpha-Isophoron, dadurch gekennzeichnet, dass alpha-Isophoron in Gegenwart eines heterogenen Katalysators in der Gasphase bei einer Temperatur von 300°C bis 400°C zu beta-Isophoron umgesetzt wird.

2. Verfahren nach Anspruch 1, dadurch gekennzeichnet, dass als Katalysator Oxide oder Mischoxide der Elemente Mg, Al, Si und Ni verwendet werden.

3. Verfahren nach Anspruch 1 oder 2, dadurch gekennzeichnet, dass als Katalysator Nickeloxid auf einem keramischen Trägermaterial eingesetzt wird.

4. Verfahren nach Anspruch 1 oder 2, dadurch gekennzeichnet, dass als Katalysator bei einer Temperatur von ≧ 1000°C calciniertes Nickeloxid auf einem γ-Aluminiumoxid-Träger eingesetzt wird.

5. Verfahren nach Anspruch 2, dadurch gekennzeichnet, dass der Katalysator Aluminiumoxid, Siliciumoxid oder Aluminiumsilikat ist.

6. Verfahren nach einem der Ansprüche 1 bis 5, dadurch gekennzeichnet, dass der Katalysator eine spezifische Oberfläche von 1-50 m²/g besitzt und einen möglichst geringen Anteil an Mikroporen und kleinen Mesoporen aufweist.

7. Verfahren nach einem der Ansprüche 1 bis 6, dadurch gekennzeichnet, dass pro Liter Katalysator und Stunde 1-10 kg alpha-Isophoron eingesetzt wird.

8. Verfahren nach einem der Ansprüche 1 bis 7, dadurch gekennzeichnet, dass die Reaktion bei einem Druck von 1 bis 200 kPa durchgeführt wird.

9. Verfahren nach einem der Ansprüche 1 bis 8, dadurch gekennzeichnet, dass die Kontaktzeit 0,1 Sekunde bis 10 Sekunden beträgt.

## Claims

1. A process for the manufacture of beta-isophorone from alpha-isophorone, characterized by converting alpha-isophorone to beta-isophorone in the presence of a heterogeneous catalyst in the gas phase at a temperature of 300°C to 400°C.

2. A process according to claim 1, characterized in that oxides or mixed oxides of the elements Mg, Al, Si and Ni are used as the catalyst.

3. A process according to claim 1 or 2, characterized in that nickel oxide on a ceramic carrier material is used as the catalyst.

4. A process according to of claim 1 or 2, characterized in that nickel oxide, calcinated at a temperature of ≥ 1000°C, on a γ-aluminium oxide carrier is used as the catalyst.

5. A process according to claim 2, characterized in that the catalyst is aluminium oxide, silicon oxide or aluminium silicate.

6. A process according to any one of claims 1 to 5, characterized in that the catalyst has a specific surface of 1-50 m²/g and an amount of micropores and small mesopores which is as low as possible.

7. A process according to any one of claims 1 to 6, characterized in that 1-10 kg of alpha-isophorone is used per litre of catalyst and hour.

8. A process according to any one of claims 1 to 7, characterized in that the reaction is carried out a pressure of 1 to 200 kPa.

9. A process according to any one of claims 1 to 8, characterized in that the contact time amounts to 0.1 second to 10 seconds.

## Revendications

1. Procédé de préparation de bêta-isophorone à partir d'alpha-isophorone, caractérisé en ce qu'on transforme l'alpha-isophorone en bêta-isophorone en présence d'un catalyseur hétérogène en phase gazeuse à une temperature de 300°C à 400°C.

2. Procédé selon la revendication 1, caractérisé en ce qu'on utilise comme catalyseur des oxydes ou oxydes mixtes des éléments Mg, Al, Si et Ni.

3. Procédé selon la revendication 1 ou 2, caractérisé en ce qu'on utilise comme catalyseur l'oxyde de nickel sur un support céramique.

4. Procédé selon la revendication 1 ou 2, caractérisé en ce qu'on utilise comme catalyseur de l'oxyde de nickel calciné à une température ≥ 1000°C sur un support de γ-oxyde d'aluminium.

5. Procédé selon la revendication 2, caractérisé en ce que le catalyseur est l'oxyde d'aluminium, l'oxyde de silicium ou le silicate d'aluminium.

6. Procédé selon l'une des revendications 1 à 5, caractérisé en ce que le catalyseur possède une surface spécifique de 1-50 m²/g et présente la plus faible proportion possible de micropores et de petits mésopores.

7. Procédé selon l'une des revendications 1 à 6, caractérisé en ce qu'on utilise par litre de catalyseur et par heure 1-10 kg d'alpha-isophorone.

8. Procédé selon l'une des revendications 1 à 7, caractérisé en ce qu'on conduit la réaction à une pression de 1 à 200 kPa.

9. Procédé selon l'une des revendications 1 à 8, caractérisé en ce que le temps de contact est compris entre 0,1 seconde et 10 secondes.
